# EUROPEAN PATENT APPLICATION

(11) **EP 3 858 280 A1**
(43) Date of publication of application: **04.08.2021**
(21) Application number: 20154360.0
(22) Date of filing: 29.01.2020
(51) Int. Cl.: A61B 34/20, G02B 27/01, A61B 90/00

(54) **SURGICAL NAVIGATION SYSTEM WITH AUGMENTED REALITY DEVICE**

(71) Applicant: Erasmus University Rotterdam Medical Center, 3015 GE Rotterdam (NL)
(72) Inventor: Benmahdjoub, Mohamed, 3015 GE Rotterdam (NL); van Walsum, Theodores, 3015 GE Rotterdam (NL)
(74) Representative: V.O.

(57) **Abstract**

A surgical navigation system (100) comprises a first detection system (10) configured to detect a first marker (11). A headset (40) comprises goggles (30) and a second detection system (20) configured to detect a distinct, second marker (21). The goggles (30) provide a display to show an augmented image (Ia) in a user's field of view (Vu). A controller (50) is configured to generate the augmented image (Ia) based at least on respective coordinates (P11,P21) of the markers (11,21), and a predetermined spatial relation (ΔP) between the coordinates (P11,P21).

## Description

### TECHNICAL FIELD AND BACKGROUND

The present disclosure relates to a surgical navigation system with an augmented reality device.

As described on *Wikipedia*, computer-assisted surgery (CAS) represents a surgical concept and set of methods, that use computer technology for surgical planning, and for guiding or performing surgical interventions. CAS is also known as computer-aided surgery, computer-assisted intervention, image-guided surgery and surgical navigation.

One component for CAS is the development of an accurate model of the patient. This can be conducted through a number of medical imaging technologies including CT, MRI, x-rays, ultrasound plus many more. For the generation of this model, the anatomical region to be operated can be scanned and uploaded into the computer system. It is possible to employ a number of scanning methods, with the datasets combined through data fusion techniques. The typical objective is the creation of a 3D dataset that reproduces the geometrical situation of the normal and pathological tissues and structures of that region. The image data taken from a patient may or may not include landmark features, in order to be able to later realign the virtual dataset against the actual patient during surgery. The landmark can also be formed by an external marker, e.g. fixed to the patient.

In computer-assisted surgery, the actual intervention can be referred to as surgical navigation. Using a surgical navigation system the surgeon may use special instruments, which can be tracked by the navigation system. The position of a tracked instrument in relation to the patient's anatomy can be shown on images of the patient, as the surgeon moves the instrument. The surgeon may thus use the system to 'navigate' an instrument to the planned location. The feedback the system provides of the instrument's location is particularly useful in situations where the surgeon cannot actually see the tip of the instrument, such as in minimally invasive surgeries.

Navigation can be improved by combining the system with an augmented reality device. For example, Kuzhagaliyev *et al.* [DOI: 10.1117/12.2293671] describe an Augmented Reality (AR) system designed to overcome challenges associated with planning and guiding the needle insertion process. The solution described in the paper is based on the HoloLens (Microsoft, USA) platform and OptiTrack (OptiTrack, USA) to track the position of the headset, needle electrodes and ultrasound (US) probe in space. The proof of concept implementation of the system uses this tracking data to render real-time holographic guides on the HoloLens, giving the user insight into the current progress of needle insertion and an indication of the target needle trajectory. The operator's field of view is augmented using visual guides and real-time US feed rendered on a holographic plane, eliminating the need to consult external monitors. Such a solution requires a calibration step between the AR device and the navigation system for each new device to be used.

There remains a need for further improvements in combining augmented reality with surgical navigation such as accurate tracking of objects in different systems.

### SUMMARY

Aspects of the present disclosure relate to a surgical navigation system and methods of integrating an augmented reality device in such system. A first detection system has a first field of view configured to detect at least a first marker. A second detection system has a second field of view configured to detect a second marker that is distinct, e.g. in nature, from the first marker. A set of goggles is provided with a display configured to show an augmented or virtual image in a user's field of view. A controller is configured to determine a first set of coordinates based on the detected first marker, determine a second set of coordinates based on the detected second marker, and generate the augmented image based at least on the first and second sets of coordinates, and a predetermined spatial relation between the first and second sets of coordinates.

Instead of adapting and calibrating the augmented reality device, a marker assembly can be constructed having trackable markers for both the first detection system as part of the navigation system and the second detection system that can be part of the augmented reality device, e.g. mounted on a headset with the goggles. When this marker assembly is seen or otherwise detected by the tracker of the navigation system and the augmented reality device, both coordinate systems can be linked (e.g. by virtue of a prior calibration). Advantageously, this can work with both optical (e.g. visible, infrared) and electromagnetic tracking (navigation systems typically use either of these for position tracking). Furthermore, the present methods can in principle work with any augmented reality device equipped with a camera and corresponding software. Additionally, it is not necessary to attach any markers to the augmented reality device.

### BRIEF DESCRIPTION OF DRAWINGS

These and other features, aspects, and advantages of the apparatus, systems and methods of the present disclosure will become better understood from the following description, appended claims, and accompanying drawing wherein:
FIG 1 illustrates aspects of a navigation system and marker assembly;
FIG 2 illustrates other or further aspects of a navigation system, and photograph of an example marker assembly;
FIGs 3A and 3B illustrate further possible embodiments of a marker assembly;
FIGs 4A - 4D illustrate a method of calibrating respective markers using optical tracking;
FIGs 5A - 5D illustrate a method of calibrating respective markers using EM tracking.

### DESCRIPTION OF EMBODIMENTS

Terminology used for describing particular embodiments is not intended to be limiting of the invention. As used herein, the singular forms "a", "an" and "the" are intended to include the plural forms as well, unless the context clearly indicates otherwise. The term "and/or" includes any and all combinations of one or more of the associated listed items. It will be understood that the terms "comprises" and/or "comprising" specify the presence of stated features but do not preclude the presence or addition of one or more other features. It will be further understood that when a particular step of a method is referred to as subsequent to another step, it can directly follow said other step or one or more intermediate steps may be carried out before carrying out the particular step, unless specified otherwise. Likewise it will be understood that when a connection between structures or components is described, this connection may be established directly or through intermediate structures or components unless specified otherwise.

The invention is described more fully hereinafter with reference to the accompanying drawings, in which embodiments of the invention are shown. In the drawings, the absolute and relative sizes of systems, components, layers, and regions may be exaggerated for clarity. Embodiments may be described with reference to schematic and/or cross-section illustrations of possibly idealized embodiments and intermediate structures of the invention. In the description and drawings, like numbers refer to like elements throughout. Relative terms as well as derivatives thereof should be construed to refer to the orientation as then described or as shown in the drawing under discussion. These relative terms are for convenience of description and do not require that the system be constructed or operated in a particular orientation unless stated otherwise.

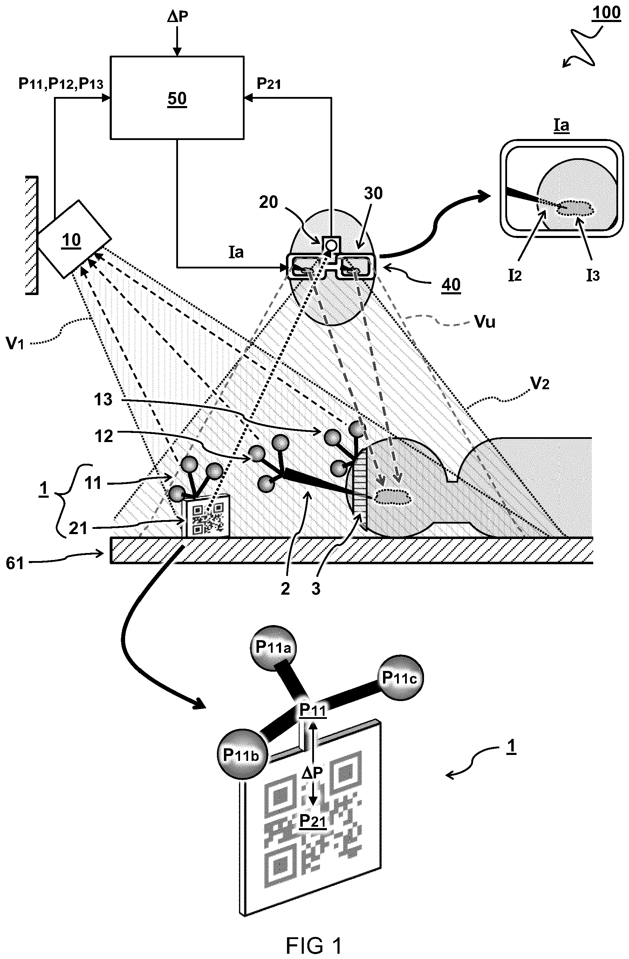

FIG 1 illustrates aspects of a navigation system 100 and marker assembly 1.

In one embodiment, the surgical navigation system 100 comprises a first detection system 10. For example, the first detection system 10 has a first field of view V1. In another or further embodiment, the first detection system is configured to detect at least a first marker 11. For example, the first marker is part of a first set of markers 11,12,13. Preferably, the navigation system comprises or is coupled to a headset 40. For example, the headset is a device typically including goggles or glasses which can be worn on the head of a user (in front of the eyes). While preferably the display is part of a headset, the present methods and systems can in principle be implemented on any mobile device, e.g. comprising the second detection system and a display configured to show an augmented image (Ia) in a user's field of view (Vu) via the display For example, instead of or in addition to the headset 40, described herein, a smartphone or tablet with a display and camera can be used. The headset or other device may be used to display augmented reality, virtual reality, mixed reality, et cetera.

In one embodiment, the headset 40 comprises a second detection system 20. For example, the second detection system 20 has a second field of view V2 which may be distinct from the first field of view V1. In another or further embodiment the second detection system 20 is configured to detect a second marker 21. This second marker 21 can be distinct from the first set of markers 11,12,13. For example, the markers are physically distinct and may also employ different modes of detection.

In one embodiment, the headset 40 comprises a set of goggles 30. Typically the headset of goggles comprises a display. Preferably, the display is configured to show an augmented image Ia in a user's field of view Vu (as seen by a user) via the goggles 30 (when wearing the headset 40). In some embodiments, the augmented image can be displayed to overlay a view through the goggles. For example, the display is at least partially transparent. Alternatively or additionally, the augmented image can be part of a recorded image that completely covers the user's field of view through the goggles. For example, the display may be opaque.

In one embodiment, the surgical navigation system 100 comprises a controller 50. In some embodiments, the controller 50 is configured to determine a first set of coordinates P11 based on the detected first marker 11. In other or further embodiments, the controller 50 is configured to determine a second set of coordinates P21 based on the detected second marker 21. In other or further embodiments, the controller 50 is configured to generate and/or position the augmented image Ia based at least on the first and second sets of coordinates P11,P21. Additionally, the augmented image Ia may be generated and/or positioned based a predetermined spatial relation ΔP between the first and second sets of coordinates P11,P21.

In one embodiment, the second detection system 20 is mounted on the headset 40 with the goggles 30. Accordingly, the second field of view V2 of the second detection system 20 can move with the user's field of view Vu as seen from a point of view of the goggles 30 (when the headset 40 is moved). For example, the second field of view V2 of the second detection system 20 substantially overlaps the user's field of view Vu. Preferably, a direction of the user's field of view Vu as seen through the goggles 30 is substantially the same as the second field of view V2 as recorded by the second detection system 20. For example, the second detection system 20 is locked in the same viewing direction as the goggles 30.

Also an extent (solid angle) of the second field of view V2 may be similar to the user's field of view Vu, e.g. within a factor 1.1, 1.2, 1.5, 2, or more. The second field of view V2 can be smaller than the user's field of view Vu, but is preferably the same or larger. Most preferably, the second detection system 20 is configured to detect objects such as the second marker 21 which can also be observed via the goggles 30. In this way, the user can be sure that the second marker 21 is used in generating the augmented image Ia with desired calibration. Additionally, or alternatively, the second field of view V2 can be relatively large to keep the second marker 21 in the second field of view V2 of the second detection system 20 even if the user is not directly looking at the second marker 21 through the goggles 30.

In one embodiment, the first detection system 10 is mounted in a position, separate and/or independent from the headset 40. During use, the position is typically stationary, although it is also possible to move the first detection system 10. For example, the first detection system 10 can determine its relative (stationary of moving) position with respect to one or more of the markers, e.g. with respect to a patient. In some embodiments the first detection system 10 can be stationary (e.g. mounted on a wall, ceiling, operating table, etc.) while the second detection system 20 is configured to move with the headset 40 according to the head of the user. For example, the first detection system 10 is mounted to have its (stationary) first field of view V1 at least partially cover a room, e.g. operating table 61. Preferably, the first field of view V1 of the first detection system 10 at least partially overlaps the second field of view V2. In this way the first marker 11 can be in the first field of view V1 while the second marker 21 (preferably rigidly interconnected in proximity to the first marker 11) can be (simultaneously) in the second field of view V2. Alternatively, or additionally, the first field of view V1 first detection system 10 can be dynamic, e.g. moveable camera following the user's field of view Vu.

While the present figure illustrates the first detection system 10 as a single unit, also additional detectors can be used as part of the first detection system 10, e.g. distributed to cover different parts of a room forming a combined or extended first field of view V1. Also, each detector may comprise multiple sensors, e.g. to produce a respective stereoscopic or higher dimensional image. So it will be understood that the first detection system 10 may comprise one or more detectors and/or sensors, e.g. disposed in different (stationary) positions and/or orientations. Similarly, the second detection system 20 may also comprise one or more detectors, preferably all mounted on the headset 40. For example, the second detection system 20 comprises a single camera; or dual cameras, one for each eye. For example, these cameras can both be normal (gray image or RGB cameras), or depth cameras, or any other camera.

Preferably, a respective set of coordinates P11,P21 of a respective marker 11,21 includes an indication of a respective position and/or orientation of the respective marker 11,21. For example, the position and orientation are determined as a respective (local) coordinate system including an origin and direction of the axes (X,Y,Z). For example, the origin and/or direction of the marker is determined with respect to a reference position and/or reference orientation.

In one embodiment, the augmented image Ia is generated based on a perspective view of a three dimensional model as seen from a point of view of the goggles 30. For example, the three dimensional model is based on three dimensional image data, which is recorded before or during operation of the system. In another or further embodiment, the augmented image Ia is generated based on a coordinate transformation between a first coordinate system and a second coordinate system. For example, the first coordinate system is associated with the surgical navigation system 100 based on the first set of coordinates P11 of the first marker 11 as detected by the first detection system 10. For example, the second coordinate system is associated with the position and orientation of the headset 40 based on the second set of coordinates P21 of the second marker 21 as detected by the second detection system 20. In some embodiments, the coordinate transformation is applied to a three-dimensional model for generating a perspective view of the model to display as the augmented image Ia.

In one embodiment, the second marker 21 is formed by a pattern with a set of distinct features which are distinguishable by the second detection system 20. For example, the second marker 21 comprises a pattern of different colors. Preferably, the pattern comprises at least three (preferably more, e.g. at least six) distinct features to allow unique recognition of the pattern, as well as determining its relative position and/or orientation. Preferably, the pattern comprises a visible pattern, e.g. the image of the second marker 21 is distinguishable in a visible wavelength range. In this way, the pattern can also be normally seen so it can be easily checked if the image is in a respective view V2 of the camera. Alternatively, or additionally, also other wavelengths such infrared can be used.

Preferably, the second set of coordinates P21 is determined based on an appearance, e.g. perspective view, of the second marker 21 as recorded by the second detection system 20. For example, the second set of coordinates P21 is determined based on a size and shape of the second marker 21 in an image recorded by the second detection system 20. In one embodiment, the second detection system 20 comprises a camera device configured to record an image including the second marker 21. Preferably, the pattern is uniquely identifiable. Accordingly, the pattern of the second marker 21 can be distinguished from other patterns in the environment. For example, a pattern of the second marker 21 can be recognized by pattern recognition in the image.

In one embodiment, the second marker 21 comprises two-dimensional pattern on a surface of a marker assembly 1. For example, the two-dimensional pattern comprises a QR or other image that is sufficiently unique and recognizable by the system. Preferably, the predetermined relative spatial relation ΔP is fixed. For example, the first marker 11 has a fixed relation to the second marker 21. Most preferably, the first marker 11 and the second marker 21 are interconnected as different parts of a single marker assembly 1. Accordingly the spatial relation between the first and second markers can be fixed and/or predetermined.

In one embodiment, the first detection system 10 comprises an infrared sensor or antenna to detect the first set of markers 11,12,13. In some embodiments, the first set of markers 11,12,13 comprise retroreflective spheres configured to reflect infrared light to the first detection system 10. For example, the first detection system 10 comprises an infrared light source (not shown) In other or further embodiments, the first set of markers 11,12,13 comprise infrared light sources detectable by the first detection system 10. For example, the first detection system 10 comprises an infrared camera. In other or further embodiments, the first set of markers 11,12,13 comprise sensor coils, e.g. for detecting radio waves and/or (alternating) magnetic fields. For example, the first detection system 10 comprises an antenna for generating an electromagnetic waves, e.g. an alternating magnetic field. These and other fields may be detectable even inside a subject. For example, an EM coil can be embedded in a tip of an instrument.

In one embodiment, the first set of markers 11,12,13 comprises a third marker (e.g. 12 or 13) detectable by the first detection system 10. In another or further embodiment, the controller 50 is configured to determine a third set of coordinates P12,P13 based on detection of the third marker 12,13. In some embodiments, the controller 50 is configured to generate the augmented image Ia including a perspective view I2,I3 of a three-dimensional model associated with a respective object 2 or subject 3 associated with the third marker 12,13. For example, the model could be an anatomical model and/or a model extended with (or replaced by) e.g. planning information (e.g. lines / guides for the surgery), et cetera. For example, generation of the augmented image Ia is based on at least the first, second, and third sets of coordinates. For example, the perspective view is determined by a coordinate transformation between the coordinate system of the surgical navigation system 100 and the coordinate system of the headset 40.

In some embodiments, the third marker is connected to an object 2, e.g. instrument, which can be manipulated by the user. For example, the system may store a three-dimensional model of the instrument which can be displayed to overlay at least part of the real instrument even when those parts are otherwise hidden from the user's field of view Vu. In other or further embodiments, the third (or fourth) marker is connected to a subject 3, e.g. fixed to a part of the body to maintain a spatial relation between its coordinates and the body part. For example, this relation can be measured before display of the augmented image Ia via an image-patient registration procedure.

Other or further aspects can be embodied as a method of displaying an augmented image Ia on a headset 40 in a surgical navigation system 100. In one embodiment, the method comprises providing a marker assembly 1 comprising a first marker 11 and a second marker 21 in a predetermined spatial relation ΔP between respective sets of coordinates of the markers 11,21. In another or further embodiment, the method comprises determining a first set of coordinates P11 based on detection of the first marker 11 by a first detection system 10 that is part of the navigation system 100. In another or further embodiment, the method comprises determining a second set of coordinates P21 based on detection of the second marker 21 by a mobile second detection system 20 that is part of the headset 40. In another or further embodiment, the method comprises generating the augmented image Ia for display on the headset 40 based at least on the first and second sets of coordinates P11,P21, and the predetermined spatial relation ΔP there between. For example, the augmented image comprises additional information I2, I3 which is otherwise not visible. In some embodiments, the position of the additional information can be determined by additional markers 12,13 and their respective coordinates. Aspects can also be embodied as a non-transitory computer-readable medium storing instructions that, when executed by one or more processors, cause performance of operational acts in accordance the methods and systems as described herein.

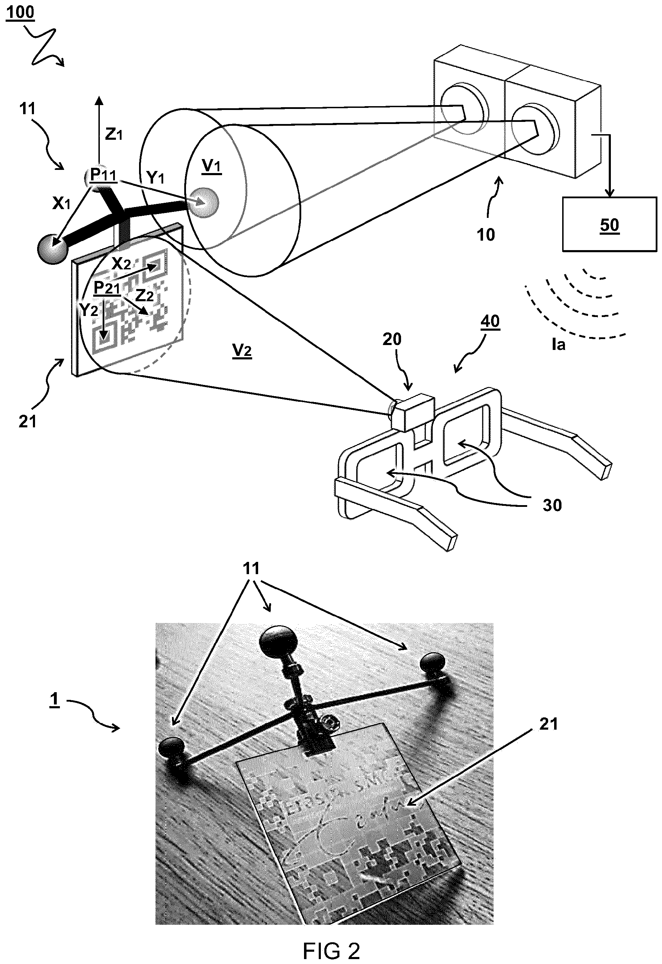

FIG 2 illustrates other or further aspects of a navigation system 100, and photograph of an example marker assembly 1. In some embodiments, e.g. as shown, the first marker 11 comprises a set of retroreflective spheres, wherein the spatial relation between the positions of the spheres can be used to determine a first coordinate system X1,Y1,Z1. For example, three or more distinct points as viewed by a (stereoscopic) camera of the first detection system 10 can be used to span a plane that can be used to determine an origin and orientation of the first marker 11. In other or further embodiments, e.g. as shown, the second marker 21 comprises a two dimensional pattern printed on a plate, wherein the extent of the pattern can be used to determine a second coordinate system X2,Y2,Z2. For example, a size and shape (perspective) of the pattern as viewed by a camera 20 of the second detection system 20 can be used to determine an angle and distance to the camera which may be related to the user's field of view through the goggles 30. In one embodiment, the controller 50 is configured to wirelessly communicate with the headset 40, e.g. for sending the augmented image Ia and/or receiving an image recorded by the second detection system 20. Also wired connections can be used.

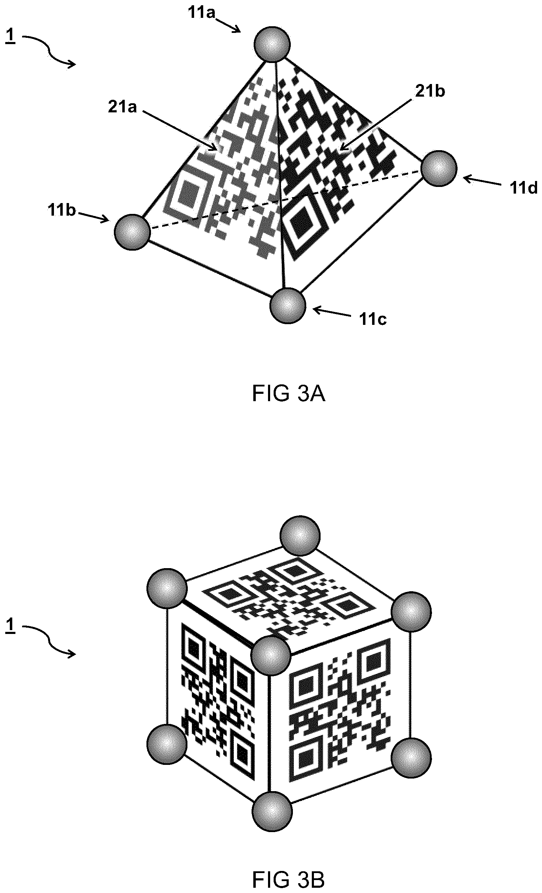

FIGs 3A and 3B illustrate further embodiments of a marker assembly 1. In one embodiment, the marker assembly 1 comprises a three-dimensional object with respective patterns 21a,21b on respective faces of the three-dimensional object forming the second marker 21 facing in different directions. By having the patterns face in different directions, this may allow the user more freedom of movement, e.g. to view the marker from different directions. For example, the three-dimensional object comprises a pyramid, cube, or any other shape allowing a perspective view of the pattern to be recognized. The pattern can be the same or different on different faces of the object. By using different patterns on different faces, this may allow further processing possibilities, e.g. determining coordinates based on two simultaneously visible different patterns.

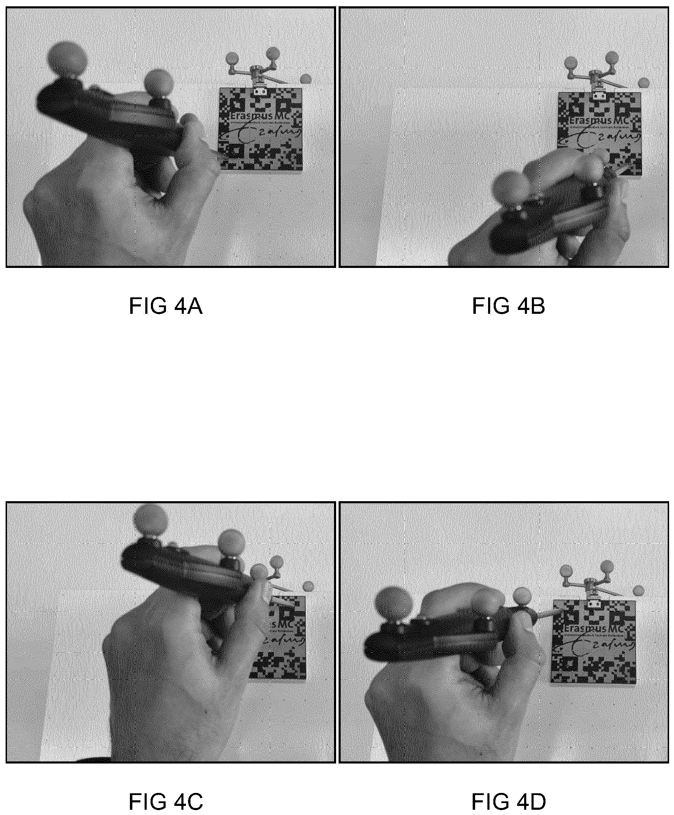

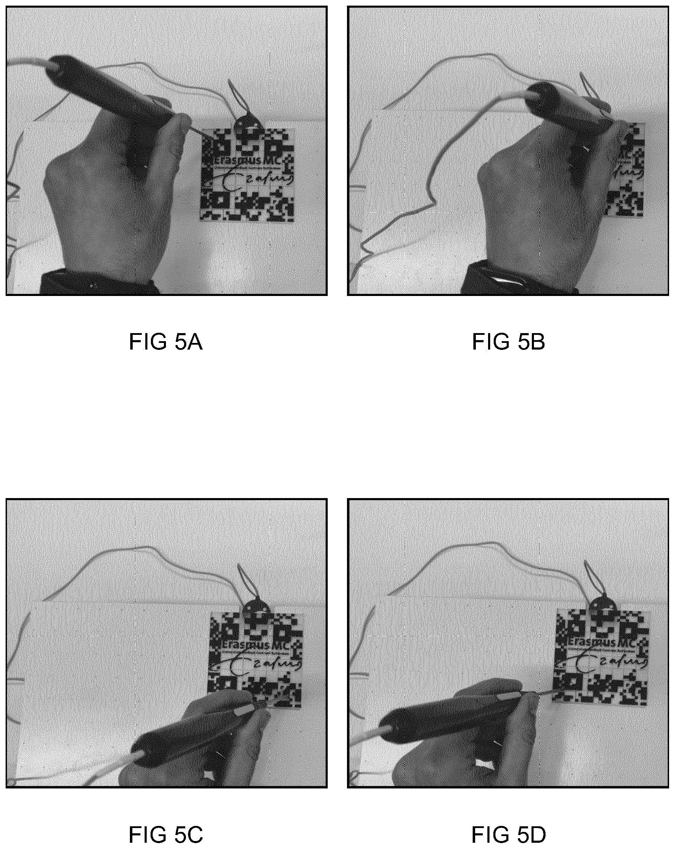

FIGs 4A - 4D illustrate a method of calibrating respective markers using optical tracking; FIGs 5A - 5D illustrate a method of calibrating respective markers using EM tracking. In some embodiments, the spatial relation is determined by a calibration relating the first set of coordinates to the second set of coordinates. For example, this can be done by overlapping respective known points of either marker. In the embodiment shown, the tip of an instrument is positioned to coincide with at least three specific points of a pattern forming the second marker. Using the three or more points of where the coordinates overlap, other coordinates can be interpolated or extrapolated, e.g. using the calibration transformation matrix.

In interpreting the appended claims, it should be understood that the word "comprising" does not exclude the presence of other elements or acts than those listed in a given claim; the word "a" or "an" preceding an element does not exclude the presence of a plurality of such elements; any reference signs in the claims do not limit their scope; several "means" may be represented by the same or different item(s) or implemented structure or function; any of the disclosed devices or portions thereof may be combined together or separated into further portions unless specifically stated otherwise. The mere fact that certain measures are recited in mutually different claims does not indicate that a combination of these measures cannot also be used to advantage. The present embodiments may thus include all working combinations of the claims wherein each claim can in principle refer to any preceding claim unless clearly excluded by context.

## Claims

1. A surgical navigation system (100) comprising
- a first detection system (10) with a first field of view (V1) configured to detect at least a first marker (11) of a first set of markers (11,12,13);
- a headset (40) comprising
∘ a second detection system (20) with a second field of view (V2) configured to detect a second marker (21) that is distinct from the first set of markers (11,12,13); and
∘ a set of goggles (30) with a display configured to show an augmented image (Ia) in a user's field of view (Vu) via the goggles (30); and
- a controller (50) configured to
∘ determine a first set of coordinates (P11) based on the detected first marker (11),
∘ determine a second set of coordinates (P21) based on the detected second marker (21), and
∘ generate the augmented image (Ia) based at least on the first and second sets of coordinates (P11,P21), and a predetermined spatial relation (ΔP) between the first and second sets of coordinates (P11,P21).

2. The system according to claim 1, wherein the second detection system (20) is mounted on the headset (40) with the goggles (30) to move the second field of view (V2) of the second detection system (20) with the user's field of view (Vu) as seen from a point of view of the goggles (30) when the headset (40) is moved.

3. The system according to any of the preceding claims, wherein the first detection system (10) is mounted in a position, separate from the headset (40).

4. The system according to any of the preceding claims, wherein a respective set of coordinates (P11,P21) of a respective marker (11,21) includes an indication of a respective position and orientation of the respective marker (11,21).

5. The system according to any of the preceding claims, wherein the augmented image (Ia) is generated based on a perspective view of a three dimensional model as seen from a point of view of the goggles (30).

6. The system according to any of the preceding claims, wherein the augmented image (Ia) is generated based on a coordinate transformation between
∘ a first coordinate system of the surgical navigation system (100) based on the first set of coordinates (P11) of the first marker (11) as detected by the first detection system (10), and
∘ a second coordinate system of the headset (40) based on the second set of coordinates (P21) of the second marker (21) as detected by the second detection system (20);
- wherein the coordinate transformation is applied to a three-dimensional model for generating a perspective view of the model to display as the augmented image (Ia).

7. The system according to any of the preceding claims, wherein the first set of markers (11,12,13) comprises a third marker (12,13) detectable by the first detection system (10), wherein the controller (50) is configured to
- determine a third set of coordinates (P12,P13) based on detection of the third marker (12,13); and
- generate the augmented image (Ia) including a perspective view (I2,I3) of a three-dimensional model associated with a respective object (2) or subject (3) associated with the third marker (12,13);
- wherein the augmented image (Ia) is based on at least the first, second, and third sets of coordinates.

8. The system according to any of the preceding claims, wherein the first detection system (10) comprises an infrared sensor or antenna to detect the first set of markers (11,12,13).

9. The system according to any of the preceding claims, wherein the second detection system (20) comprises a camera device configured to record an image including a perspective view of the second marker (21).

10. The system according to any of the preceding claims, wherein the second marker (21) is formed by a two-dimensional pattern with a set of distinct features which are distinguishable by the second detection system (20), wherein the second set of coordinates (P21) is determined based on a size and shape of the second marker (21) in an image recorded by the second detection system (20).

11. The system according to any of the preceding claims, wherein the first marker (11) and the second marker (21) are interconnected as different parts of a single marker assembly (1).

12. The system according to any of the preceding claims, wherein the second marker (21) comprises a two-dimensional pattern such as a QR code on a surface of a marker assembly (1).

13. The system according to claim 12, wherein the marker assembly (1) comprises a three-dimensional object with multiple patterns (21a,21b) on respective faces of the three-dimensional object forming the second marker (21) facing in different directions.

14. A method of displaying an augmented image (Ia) on a headset (40) in a surgical navigation system (100), the method comprising
- providing a marker assembly (1) comprising a first marker (11) and a second marker (21) in a predetermined spatial relation (ΔP) between respective sets of coordinates of the markers (11,21);
- determining a first set of coordinates (P11) based on detection of the first marker (11) by a first detection system (10) that is part of the navigation system (100);
- determining a second set of coordinates (P21) based on detection of the second marker (21) by a mobile second detection system (20) that is part of the headset (40); and
- generating the augmented image (Ia) for display on the headset (40) based at least on the first and second sets of coordinates (P11,P21), and the predetermined spatial relation (ΔP) there between.

15. A non-transitory computer-readable medium storing instructions that, when executed by one or more processors, cause a device to perform the method according to claim 14.
